# EUROPEAN PATENT SPECIFICATION

(11) **EP 4 005 516 B1**
(45) Date of publication and mention of the grant of the patent: **08.01.2025**
(21) Application number: 21210100.0
(22) Date of filing: 24.11.2021
(51) Int. Cl.: A61B 18/12, A61B 18/00

(54) **SINGLE FREQUENCY SWITCH MODE POWER SUPPLY GENERATOR WITH PHASE SHIFTER**
EINZELFREQENZ-STROMVERSORGUNGSGENERATOR MIT PHASENSCHIEBER
GÉNÉRATEUR D'ALIMENTATION ÉLECTRIQUE DE FRÉQUENCE UNIQUE AVEC DÉPHASEUR

(30) Priority: 25.11.2020 US 202017104823
(43) Date of publication of application: 01.06.2022
(73) Proprietor: Biosense Webster (Israel) Ltd., Yokneam 2066717 (IL)
(72) Inventor: ALTMANN, Andres Claudio, 2066717 Yokneam (IL); GOVARI, Assaf, 2066717 Yokneam (IL); KOPAIGORODSKI, Alexander, 2066717 Yokneam (IL)
(74) Representative: Carpmaels & Ransford LLP

(56) References cited:
- US-A- 4 727 874
- US-A1- 2003 199 868
- US-A1- 2011 071 518
- US-A1- 2019 069 943

## Description

### BACKGROUND

Conventional ablation methods and systems, such as radio-frequency (RF) catheter ablation, are used to ablate portions of dysfunctional tissue, such as tissue of a heart, lung, ear, nose, throat or other organs of patient anatomy. For example, during a medical procedure, such as an RF catheter ablation procedure, a catheter is typically inserted through an incision in the skin and guiding the catheter to an organ where the catheter is used to create ablation lesions on the organ tissue.

The location of the medical tool in three-dimensional (3-D) space within patient anatomy is determined using electromagnetic navigation, which includes electromagnetic emitters and electromagnetic sensors on the tool to determine the tool's location. Based on the determined location, anatomical information of the patient is displayed to medical personnel. Dynamic maps of the patient anatomy (e.g., organs) are created to facilitate accurate determination of regions for ablation. Target ablation sites (i.e., regions of interest (ROI)) of an organ are identified by viewing the maps. Based on the identified ablation sites, an ablation procedure, which includes one or more ablations, is performed on the organ.
US2011071518A1 describes a system and method for multi-pole phase-shifted radio frequency application in which an electrosurgical generator is disclosed. The generator includes a power supply operable to generate a DC voltage and a multi-pole, phase-shifted, pulse-width and/or frequency modulated RF output stage coupled to the power supply. The RF output stage includes a plurality of dual-pole circuits, each of the plurality of dual-pole circuits including first and second pairs of switching components. The generator also includes a controller configured to drive the first and second pairs of switching components of each of the plurality of dual-pole circuits at a predetermined phase-shifted frequency. US 2019/069943 discloses a signal generator, control circuitry, a plurality of non-linear amplifiers, and a processor. The signal generator is configured to generate an RF signal having a given frequency. The control circuitry is configured to set phases and amplitudes of a plurality of replicas of the RF signal generated by the signal generator. The plurality of non-linear amplifiers is configured to amplify the plurality of replicas of the RF signal, and to drive a respective plurality of ablation electrodes in a patient body with the amplified replicas. The processor is configured to receive a return signal, including a superposition of the replicas sensed by a patch electrode attached to the patient body, and to adaptively adjust the phases and amplitudes of the replicas in response to the return signal, by controlling the control circuitry.

### BRIEF DESCRIPTION OF THE DRAWINGS

A more detailed understanding can be had from the following description, given by way of example in conjunction with the accompanying drawings wherein:
FIG. 1 is an illustration of an example medical system for navigating a tool in 3-D space according to embodiments disclosed herein;
FIG. 2 is an illustration of components of an example electromagnetic navigation system for use with embodiments described herein;
FIG. 3 is a diagram of components of a medical tool, including a switch mode power supply generator which operates on a signal frequency, which can be used to implement features of the present disclosure; and
FIG. 4 is a diagram of components of a medical tool, including a Class D inverter power amplifier, which can be used to implement features of the present disclosure.
FIG. 5 is a flow diagram illustrating an example method of controlling power supplied for a medical ablation procedure.

### DETAILED DESCRIPTION

The methods and systems conventionally used to identify ablation sites and perform the ablation procedure are time consuming (e.g., several hours) and rely on medical personnel with specific expertise and experience (typically requiring many hours of training). Successful treatment depends on accurate identification of ablation sites as well as an accurate assessment of the ablations performed on the organ.

Typically, ablation is performed by applying RF energy to an organ via multiple electrodes of a medical tool, such as a catheter. Radiofrequency (RF) generators are used to supply power to the electrodes of the medical tool for the ablation.

Conventional RF generators include linear power supplies which apply AC voltage to a transformer to change (e.g., step down) the voltage before being applied to a regulator to regulate the voltage. The components (e.g., transformer and regulator circuitry) of these conventional linear power supplies used to regulate the voltage are relatively large and generate more heat, resulting in lower energy efficiency. For example, conventional linear power supply generators have limited power yield (e.g., 25 watts of RF energy) and require high amounts of power to ablate.

In addition, for multi-catheter ablation, conventional RF generators control the power supplied to each electrode by driving the electrodes with signals having distinct frequencies, resulting in the presence of intermodulation distortion (IMD). IMD occurs when multiple signals of different frequencies are mixed together and additional signals are formed at frequencies that are not, in general, at harmonic frequencies of either signal. Accordingly, the IMD can cause the ablation signals to interfere with other signals, such as electrocardiogram (ECG) signals.

The invention provides a power generator according to claim 1. Embodiment of the invention are defined in the dependent claims. The present application further provides a medical tool, such as a catheter, which includes a switched-mode power supply generator that operates on a single frequency. The power generator includes a plurality of switched-mode type amplifiers, each electrically connected to one of a plurality of ablation electrodes of a medical tool, such as a catheter. In contrast to conventional linear power supply RF generators, the switched-mode power supply RF generator converts the AC power directly into a DC voltage without the use of a transformer. Accordingly, the switched-mode power supply generator is smaller and more efficient (e.g., higher power conversion ratio) than conventional linear power supply generators used for RF ablation.

In addition, the switched-mode power supply generators disclosed herein control the supply power to the electrodes by an applied voltage and phase shift. That is, the amplitude is controlled for the signals output to each of the electrodes at the same frequency but with different phase shifts to control the power yielded by each electrode. Because the power is controlled using a single frequency, IMD is not present and the ablation signals do not interfere with other signals.

The present application discloses a system used to perform a medical ablation procedure. The system comprises a medical tool comprising a plurality of electrodes used to apply radio frequency (RF) energy for ablating tissue and a power generator a power supply configured to generate a DC voltage. The power generator comprises a power supply configured to generate a DC voltage, a phase shifter configured to shift signal transmission phase angles and a plurality of switched-mode amplifiers each electrically connected to a corresponding one of the electrodes and each configured to convert the DC voltage received from the power supply to an AC voltage signal provided to the corresponding electrode. The power generator also comprises a processor configured to control the power yielded by each electrode by controlling a phase shift of each AC voltage signal converted by the switched-mode amplifiers and controlling amplitude of each AC voltage signal convert by the switched-mode amplifiers.

The present application discloses a method of controlling power supplied for a medical ablation procedure. The method comprises supplying a DC voltage, converting, by each one of a plurality of switched-mode amplifiers, the DC voltage to a corresponding AC voltage signal, controlling a phase shift of each AC voltage signal converted by the switched-mode amplifiers and controlling an amplitude of each AC voltage signal convert by the switched-mode amplifiers. Although the method is not claimed, it may be used with the power generator of the present invention, and is considered useful for understanding the invention.

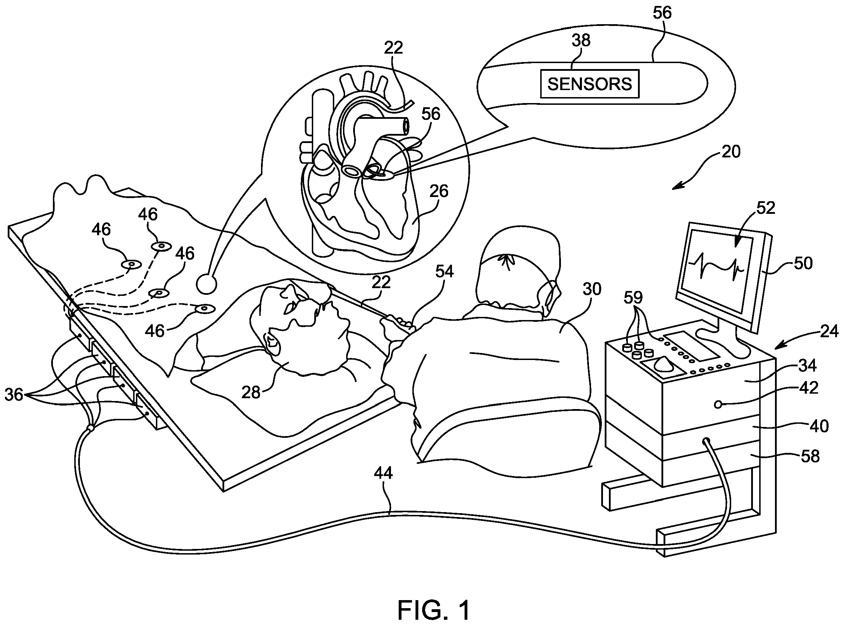

Referring now to FIG. 1, an illustration of an example medical system 20 is shown that may be used to generate and display information 52 (e.g., a chart, anatomical models of a portion of a patient and signal information). Tools (i.e., medical tools), such as tool 22, can be any tool which includes a catheter and a sheath (e.g., steerable and deflectable sheath) used for diagnostic or therapeutic treatment, such as for mapping electrical potentials in a heart 26 of a patient 28. Alternatively, tools may be used, mutatis mutandis, for other therapeutic and/or diagnostic purposes of different portions of anatomy, such as in the heart, lungs or other body organs, such as the ear, nose, and throat (ENT). Tools may include, for example, probes, catheters, cutting tools and suction devices.

An operator 30 may insert the tool 22 into a portion of patient anatomy, such as the vascular system of the patient 28 so that a tip 56 of the tool 22 enters a chamber of the heart 26. The control console 24 may use magnetic position sensing to determine position coordinates of the tool (e.g., coordinates of the tip 56) in 3-D space inside the heart 26. As described in more detail below, magnetic position sensing is used to determine the location of both a catheter and a sheath of the tool 22 in 3-D space. To determine the position coordinates, a driver circuit 34 in the control console 24 may drive, via connector, 44, field generators 36 to generate magnetic fields within the anatomy of the patient 28.

The field generators 36 include one or more emitter coils (not shown in FIG. 1), placed at known positions external to the patient 28, which are configured to generate magnetic fields in a predefined working volume that contains a portion of interest of the patient anatomy. Each of the emitting coils is driven by a different frequency to emit a constant magnetic field in 3-D space. For example, in the example medical system 20 shown in FIG. 1, one or more emitter coils can be placed below the torso of the patient 28 and each configured to generate magnetic fields in a predefined working volume that contains the heart 26 of the patient.

One or more electromagnetic sensors (i.e., magnetic field location sensors) 38 are disposed on a catheter of the tool 22 which generate electrical signals based on the amplitude and phase of the magnetic fields to determine the position of the catheter in 3-D space. As described in more detail below, three separate electromagnetic sensors 38 are also disposed on a sheath of the tool 22 to accurately determine the location of the sheath using electromagnetic based navigation.

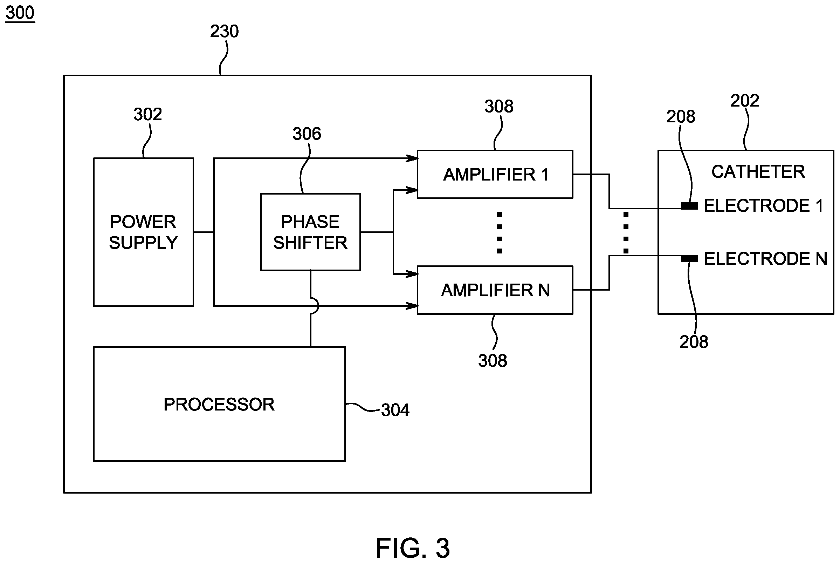

The signals are wirelessly communicated to the control console 24 via a wireless communication interface (e.g., interface 312 shown at FIG. 3) at the tool 22 that may communicate with a corresponding input/output (I/O) interface 42 in the control console 24. The wireless communication interface 312 and the I/O interface 42 may operate in accordance with any suitable wireless communication standard that is known in the art, such as for example, infrared (IR), radio frequency (RF), Bluetooth, one of the IEEE 802.11 family of standards (e.g., Wi-Fi), or the HiperLAN standard. The body surface electrodes 46 may include one or more wireless sensor nodes integrated on a flexible substrate. The one or more wireless sensor nodes may include a wireless transmit/receive unit (WTRU) enabling local digital signal processing, a radio link, and a miniaturized rechargeable battery, as described in more detail below.

The I/O interface 42 may enable the control console 24 to interact with the tool 22, the body surface electrodes 46 and the position sensors (not shown). Based on the electrical impulses received from the body surface electrodes 46 and the electrical signals received from the tool 22 via the I/O interface 42 and other components of medical system 20, the signal processor 40 may determine the locations of the catheter and sheath of the tool 22 in 3-D space and generate the display information 52, which may be shown on a display 50.

The signal processor 40 is configured to process the signals to determine the position coordinates of both the catheter and sheath of the tool 22 in 3-D space, including both location and orientation coordinates. Each of the magnetic field location sensors 38 transmit a signal to the control console 24 which indicates location coordinates of the tool 22 (e.g., location coordinates of the catheter and sheath of the tool 22) in 3-D space.

The signal processor 40 may be included in a general-purpose computer, with a suitable front end and interface circuits for receiving signals from the tool 22 and controlling the other components of the control console 24. The signal processor 40 may be programmed, using software, to perform the functions that are described herein. The software may be downloaded to the control console 24 in electronic form, over a network, for example, or it may be provided on non-transitory tangible media, such as optical, magnetic or electronic memory media. Alternatively, some or all of the functions of the signal processor 40 may be performed by dedicated or programmable digital hardware components.

In the example shown at FIG. 1, the control console 24 is connected, via cable 44, to body surface electrodes 46, each of which are attached to patient 28 using patches (e.g., indicated in FIG. 1 as circles around the electrodes 46) that adhere to the skin of the patient. In addition or alternative to the patches, body surface electrodes 46 may also be positioned on the patient using articles worn by patient 28 which include the body surface electrodes 46 and may also include one or more position sensors (not shown) indicating the location of the worn article. For example, body surface electrodes 46 can be embedded in a vest that is configured to be worn by the patient 28. During operation, the body surface electrodes 46 assist in providing a location of the tool (e.g., catheter) in 3-D space by detecting electrical impulses generated by the polarization and depolarization of cardiac tissue and transmitting information to the control console 24, via the cable 44. The body surface electrodes 46 can be equipped with magnetic location tracking and can help identify and track the respiration cycle of the patient 28.

Additionally or alternatively, the tool 22, the body surface electrodes 46 and other sensors (not shown) may communicate with the control console 24 and one another via a wireless interface. For example, a wireless catheter, which is not physically connected to signal processing and/or computing apparatus, can communicate with the control console 24. For example, a transmitter/receiver is attached to the catheter which communicates with a signal processing and/or computer apparatus using wireless communication methods, such as IR, RF, Bluetooth, or acoustic transmissions.

During the diagnostic treatment, the signal processor 40 may present the display information 52 and may store data representing the information 52 in a memory 58. The memory 58 may include any suitable volatile and/or non-volatile memory, such as random access memory or a hard disk drive. The operator 30 may be able to manipulate the display information 52 using one or more input devices 59. Alternatively, the medical system 20 may include a second operator that manipulates the control console 24 while the operator 30 manipulates the tool 22. It should be noted that the configuration shown in FIG. 1 is exemplary. Any suitable configuration of the medical system 20 may be used and implemented.

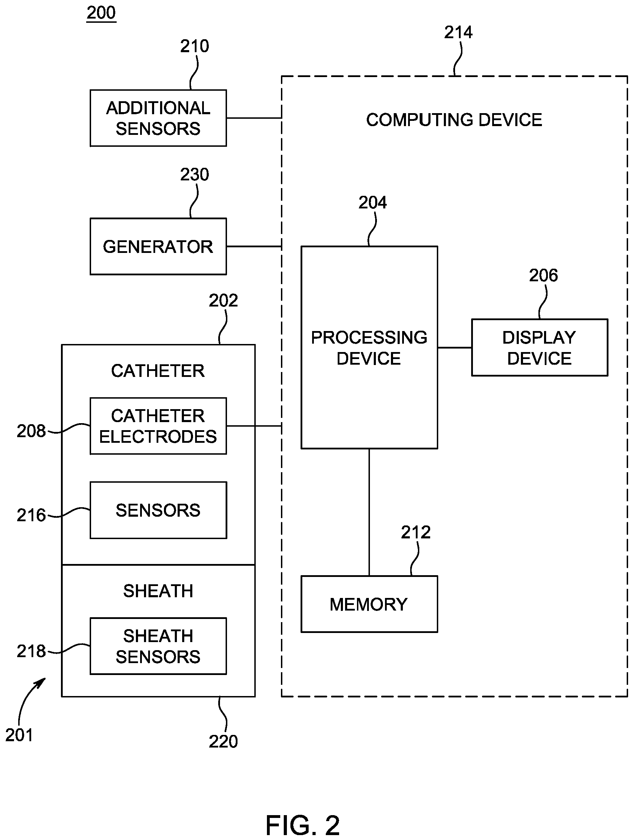

FIG. 2 is a block diagram illustrating example components of a medical system 200 for use with embodiments described herein. As shown in FIG. 2, the system 200 includes a medical tool 201, a processing device 204, a display device 206 and memory 212. The medical tool 201 includes a catheter 202 and a sheath 220. The catheter 202 is, for example, an ablation catheter used to ablate portions (e.g., tissue) in patient anatomy. The sheath 220 is, for example, steerable and deflectable to facilitate, for example, catheter access, stability, and tissue contact in target sites within patient anatomy. For example, during operation the catheter 202 is guided within patient anatomy (e.g., via a blood vessel) through the steerable and deflectable sheath 220 to a target location (e.g., a heart).

As shown in FIG. 2, the processing device 204, display device 206 and memory 212 are a part of an example computing device 214. In some embodiments, the display device 206 may be separate from computing device 214. Computing device 214 may also include an I/O interface, such as I/O interface 42 shown in FIG. 1.

As shown in FIG. 2, the example catheter 202 includes one or more sensors 216, which include, for example, a magnetic field location sensor (e.g., sensor 38 in FIG. 1) for providing location signals to indicate the 3-D position coordinates of the catheter 202. Likewise, the example sheath 220 includes one or more sensors 218, which include, for example, a magnetic field location sensor for providing location signals to indicate the 3-D position coordinates of the sheath 220. In some procedures, one or more additional sensors 210 that are separate from the catheter 202, as shown in example system 200, are also used to provide location signals. In some embodiments, the catheter 202 also includes catheter electrodes 208 for mapping electrical potentials of a heart.

Sensors 216 (and 218) may also include, for example, position sensors, pressure or force sensors, temperature sensors, impedance sensors or other sensors which provide ablation parameter signals indicating ablation parameters during the ablation of tissue of an organ. During the ablation procedure, RF generator 230 supplies high-frequency electrical energy, via catheter 202, for ablating tissue at locations engaged by the catheter 202. Sensors 216, 218 sense ablation parameters (e.g., catheter 202 or sheath 220 position stability, temperature, ablation time, ablation power and ablation impedance) during the ablation procedure. Catheter 202 or sheath 220 may be in wired or wireless communication with processing device 204 to communicate the information acquired by sensors 216, 218.

The location signals are processed as location data and stored, for example, in memory 212. The processing device 204 receives (e.g., reads from memory) location data corresponding to the location signals and generates mapping information, from the location data, for displaying one or more maps of an organ being ablated. The ablation parameter signals are processed as ablation parameter data and stored, for example, in memory 212.

The processing device 204 receives the ablation parameter data corresponding to the ablation parameter signals and generates, from the ablation parameter data, first object information for displaying a first geometrical object having a first size which represents an estimated depth of the ablation of the organ. Processing device 204 also receives, from the ablation parameter data, second object information for displaying, concurrently with the first geometrical object, a second geometrical object having a second size which represents an estimated width of the ablation of the organ.

That is, the processing device 204 receives the ablation parameter data corresponding to ablation parameter signals acquired (e.g., via one or more sensors 216) during the ablation procedure, determines from the ablation parameter data, estimated depth and width of an ablation, and generates, from the ablation parameter data, object information for displaying geometric objects to visually represent the estimated ablation depth and width. For example, using the ablation parameter data, processing device 204 executes a plurality of programmed instructions (e.g., lesion estimation and assessment algorithms) to determine an estimated depth and width of an ablation. The processing device 204 then generates first object information for displaying a first geometrical object having a first size which represents the estimated depth for an ablation of the heart. The processing device 204 also generates second object information for displaying, concurrently with the first geometrical object, a second geometrical object having a second size which represents the estimated width for the ablation of the heart.

The processing device 204 may also use the ablation parameter data to execute the programmed instructions to generate in-blood information for displaying an indicator on the map of an organ to visually represent a portion of the organ tissue which was not contacted during the ablation procedure. For example, during the ablation procedure, ablation parameter signals may be acquired, via sensors 216, indicating whether the catheter 202 contacts the organ tissue at a portion of the heart. The ablation parameter signals may include, for example, information identifying the location of the catheter in 3-D space at a particular time, information identifying a force applied by the catheter, impedance information and other information indicating whether the catheter 202 contacts the organ tissue at the portion of the organ.

The processing device 204 processes the ablation parameter signals as ablation parameter data and uses the ablation parameter data to determine whether the catheter 202 contacted the organ tissue at the portion of the organ. If no contact is determined between the ablation device and the heart tissue at the portion of the organ, the processing device 204 generates in-blood indicator information, indicating an in-blood ablation (as opposed to an ablation of the organ tissue).

Processing device 204 drives display device 206, using the mapping information, to display the map of the organ on display device 206. Processing device 204 also drives display device 206, using the first object information and the second object information, to display the first and second geometrical objects at the display device 206 as well as any determined in-blood indicators.

Display device 206 may include one or more displays each configured to display one or more maps of the organ. For example, display device 206 is configured to display maps representing a spatio-temporal manifestation of an organ (e.g., a heart) as well as geometrical objects which represent estimated ablation depths and widths. Display device 206 may be in wired or wireless communication with processing device 204. In some embodiments, display device may be separate from computing device 214.

Memory 212 includes, for example, volatile and non-volatile memory, such as random access memory (RAM), dynamic RAM, or a cache. Memory 212 also includes, for example, storage, such as, fixed storage (e.g., a hard disk drive and a solid state drive) and removable storage (e.g., an optical disk and a flash drive).

As shown in FIG. 3, the system 200 also includes switch mode power supply generator 230. The generator 230 is for example, in communication (e.g., wired or wireless communication) with computing device 214 (e.g., processing device 204 of computing device 214). Generator 230 is configured to supply power to each of electrodes 208 for performing ablation. Generator 230 is described in more detail below with regard to FIG. 3.

FIG. 3 is a diagram of components of an example medical system 300 for use during an ablation procedure. As shown in FIG. 3, the medical system 300 includes switch mode power supply generator 230 and catheter 202. The switch mode power supply generator 230 includes power supply (e.g., battery) 302, processor 304, phase shifter 306, which can be implemented as hardware (e.g., voltage control phase shifter), software or a combination of hardware and software to shift transmission phase angles of signals, and amplifiers 308. Each one of amplifiers 308 is electrically connected to one of the electrodes 208 of catheter 202.

Processor 304 is configured to control the amount of power yielded by each electrode 208 via the phase shifter 306 and amplifiers 308. Processor 304 communicates with each amplifier 308 to control the amplitude of the output signal and the phase in which the signal is provided to each electrode 208. The signals for each electrode 208 are sent using the same frequency, but at different phases. That is, processor 304 controls the phase shift of the different signals such that each signal to one of the electrodes is identified via its corresponding phase. For example, if the catheter 202 includes 10 electrodes, then 10 different signals are sent using the same frequency (e.g., 480kHz) but at different phases. Because power is controlled using a single frequency, intermodulation is not present and the ablation signals do not interfere with the ECG signals.

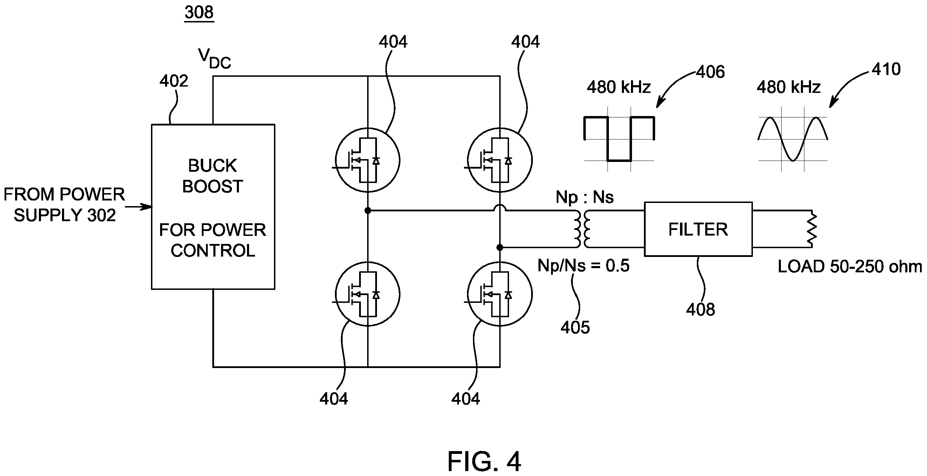

FIG. 4 is a diagram illustrating example components of an amplifier 308 shown in FIG. 3. As shown in FIG. 4, the amplifier 400 includes a Buck Boost converter 402, two pairs of N-channel metal-oxide-semiconductor field-effect (MOSFET) transistors 404 and filter 408.

The Buck Boost converter 402 is a DC-to-DC power converter, which is controlled by processor 304 to step up and step down an input DC voltage and facilitate power control. The Buck Boost converter 402 receives a DC voltage signal from power supply 302 and regulates the DC voltage provided to the MOSFET transistors 404.

The N-channel MOSFET transistors 404 are controlled, by processor 304, to switch between different states to convert the DC signal into amplitude pulses. These pulses are amplified via transformer 405 to provide the digital AC voltage signal 406 in the form of square wave (e.g., at a frequency of 480kHz).

Although the transistors 404 shown in FIG. 4 include 2 pairs of N-channel MOSFET transistors, the number and type of transistors shown in FIG. 4 is merely an example. Amplifiers can also include other types of semiconductors or switching components to implement features of the present disclosure. The transformer 405 shown in FIG. 4 includes a turn ratio (number of primary windings Np/number of secondary windings Ns) equal to 0.5. The turn ratio is merely an example. Amplifiers can include transformers having different turn ratios to implement the features disclosed herein. Additionally, although MOSFET transistors are set forth in the example of FIG. 4, IGBT transistors may be utilized in accordance with the present teachings.

The filter 408 is, for example, a low pass filter which is configured to convert the AC voltage signal 406 to an analog AC voltage signal 410 delivered to one of the electrodes 208 shown in FIG. 3. As shown in FIG. 4, the square wave AC voltage signal 406, having a frequency of 480 kHz, is converted to the analog sinusoidal AC voltage signal 410, having the frequency of 480 kHz, which is delivered to one of the electrodes 208. The filter 408 is, for example, a low pass filter such as a Butterworth filter and may include an inductor, a capacitor or other electrical components which can be used to attenuate the high frequency switching components and pass the frequency band (e.g., band which includes 480 kHz).

Because the switched-mode power supply RF generator 230 includes a plurality of switched-mode type amplifiers 308, the switched-mode power supply generator 308 is smaller and more efficient (e.g., higher power conversion ratio) than conventional linear power supply generators used for RF ablation.

In addition, because the switched-mode power supply RF generator 230 controls the supply power to the electrodes by an applied voltage and phase shift, the power supplied to each of the electrodes 208 is controlled using a single frequency. Accordingly, the power supply signals provided to each of the electrodes 208 do not interfere with other signals (e.g., ECG signals) because the IMD that is present with conventional power supply generators is avoided.

It should be noted that although switched-mode amplifiers 308 are set in FIG. 3, one or more linear power amplifiers can be utilized in an alternative embodiment. Such operational amplifiers are well known to those of skill in the art.

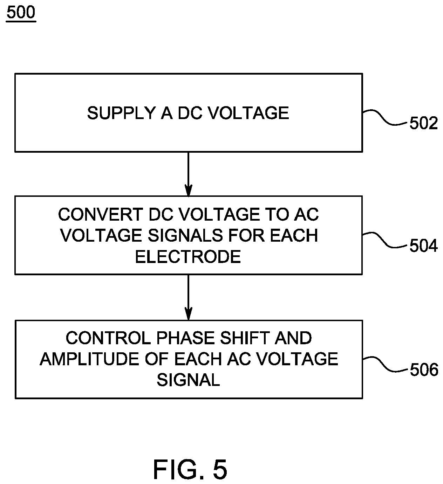

FIG. 5 is a flow diagram illustrating an example method 500 of controlling power supplied for a medical ablation procedure.

As shown at block 502, the method 500 includes supplying a DC voltage. For example, the DC voltage is supplied via a battery of a RF switched mode power supply generator.

As shown at block 504, the method 500 includes converting, via each of a plurality of switched-mode amplifiers, the DC voltage to a corresponding AC voltage signal.

As shown at block 506, the method 500 includes controlling a phase shift and amplitude of each AC voltage signal converted by the switched-mode amplifiers.

The methods provided herein may be implemented in a general purpose computer, a processor, or a processor core. Suitable processors include, by way of example, a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs) circuits, any other type of integrated circuit (IC), and/or a state machine. Such processors can be manufactured by configuring a manufacturing process using the results of processed hardware description language (HDL) instructions and other intermediary data including netlists (such instructions capable of being stored on a computer readable media). The results of such processing can be maskworks that are then used in a semiconductor manufacturing process to manufacture a processor which implements features of the disclosure.

The methods or flow charts provided herein can be implemented in a computer program, software, or firmware incorporated in a non-transitory computer-readable storage medium for execution by a general purpose computer or a processor. Examples of non-transitory computer-readable storage mediums include a read only memory (ROM), a random access memory (RAM), a register, cache memory, semiconductor memory devices, magnetic media such as internal hard disks and removable disks, magneto-optical media, and optical media such as CD-ROM disks, and digital versatile disks (DVDs).

It should be understood that many variations are possible based on the disclosure herein. The scope of the invention is defined by the appended claims.

## Claims

1. A power generator (230) for use with a medical tool (202) used to perform a medical ablation procedure comprising:
a power supply (302) configured to generate a DC voltage;
a phase shifter (306) configured to shift signal transmission phase angles;
a plurality of switched-mode amplifiers (308) each comprising N-channel metal-oxide-semiconductor field-effect (MOSFET) transistors (404), and each configured to convert the DC voltage received from the power supply to an AC voltage signal; and
a processor (304) configured to control:
the phase shifter (306) to control a phase shift of each AC voltage signal converted by the switched-mode amplifiers;
an amplitude of each AC voltage signal converted by the switched-mode amplifiers; and
the MOSFET transistors to switch between different states to convert the DC voltage into amplitude pulses.

2. A system (300) configured to perform a medical ablation procedure comprising:
A medical tool (202) comprising a plurality of electrodes (208) used to apply radio frequency (RF) energy for ablating tissue; and
a power generator according to claim 1,
wherein each of the plurality of switched-mode amplifiers is electrically connected to a corresponding one of the plurality of electrodes and wherein the AC voltage signal of each switched-mode amplifier is provided to the corresponding one of the plurality of electrodes; and
wherein the processor is configured to control the power yielded by each electrode.

3. The system of claim 2, wherein the medical tool is a catheter.

4. The power generator of claim 1 or the system of claim 2, wherein each AC voltage signal is provided at a same frequency.

5. The system of claim 2, wherein the amplitude of each AC voltage signal corresponds to the power yielded by each electrode.

## Patentansprüche

1. Stromgenerator (230) zur Verwendung mit einem medizinischen Instrument (202), das verwendet wird, um ein medizinisches Ablationsverfahren durchzuführen, umfassend:
eine Stromversorgung (302), die konfiguriert ist, um eine Gleichspannung zu erzeugen;
einen Phasenschieber (306), der konfiguriert ist, um Signalübertragungs-Phasenwinkel zu verschieben;
eine Vielzahl von Schaltverstärkern (308), die jeweils N-Kanal-Metalloxid-Halbleiter-Feldeffekttransistoren (MOSFET-Transistoren) (404) umfassen und jeweils konfiguriert sind, um die Gleichspannung, die von der Stromversorgung empfangen wird, in ein Wechselspannungssignal umzuwandeln; und
einen Prozessor (304), der konfiguriert ist zum Steuern von Folgendem:
dem Phasenschieber (306), um eine Phasenverschiebung von jedem Wechselspannungssignal, das durch die Schaltverstärker umgewandelt wird, zu steuern;
einer Amplitude von jedem Wechselspannungssignal, das durch die Schaltverstärker umgewandelt wird; und
die MOSFET-Transistoren zwischen unterschiedlichen Zuständen wechseln, um die Gleichspannung in Amplitudenimpulse umzuwandeln.

2. System (300), das konfiguriert ist, um ein medizinisches Ablationsverfahren durchzuführen, umfassend:
Medizinisches Instrument (202), umfassend eine Vielzahl von Elektroden (208), die verwendet werden, um Hochfrequenzenergie (HF-Energie) für die Ablation von Gewebe anzulegen; und
einen Stromgenerator nach Anspruch 1,
wobei jeder der Vielzahl von Schaltverstärkern mit einer entsprechenden der Vielzahl von Elektroden elektrisch verbunden ist, und wobei das Wechselspannungssignal von jedem Schaltverstärker an die entsprechende der Vielzahl von Elektroden bereitgestellt wird; und
wobei der Prozessor konfiguriert ist, um den Strom, der durch jede Elektrode erbracht wird, zu steuern.

3. System nach Anspruch 2, wobei das medizinische Instrument ein Katheter ist.

4. Stromgenerator nach Anspruch 1 oder das System nach Anspruch 2, wobei jedes Wechselspannungssignal mit der gleichen Frequenz bereitgestellt wird.

5. System nach Anspruch 2, wobei die Amplitude von jedem Wechselspannungssignal dem Strom entspricht, der durch jede Elektrode erbracht wird.

## Revendications

1. Générateur d'énergie (230) à utiliser avec un outil médical (202) utilisé pour effectuer une procédure d'ablation médicale comprenant :
une alimentation électrique (302) configurée pour générer une tension continue ;
un déphaseur (306) configuré pour décaler les angles de phase de la transmission du signal ;
une pluralité d'amplificateurs à mode commuté (308) comprenant chacun des transistors à effet de champ à métal-oxyde-semiconducteur (MOSFET) à canal N (404), et chacun configurés pour convertir la tension continue reçue de l'alimentation électrique en un signal de tension alternative ; et
un processeur (304) configuré pour contrôler :
le déphaseur (306) pour contrôler un déphasage de chaque signal de tension alternative converti par les amplificateurs à découpage ;
une amplitude de chaque signal de tension alternative converti par les amplificateurs à découpage ; et
les transistors MOSFET passent d'un état à l'autre pour convertir la tension continue en impulsions d'amplitude.

2. Système (300) configuré pour effectuer une procédure d'ablation médicale comprenant :
un outil médical (202) comprenant une pluralité d'électrodes (208) utilisées pour appliquer une énergie de radiofréquence (RF) afin d'ablater un tissu ; et
un générateur d'énergie selon la revendication 1,
dans lequel chacun des amplificateurs à découpage est connecté électriquement à une électrode correspondante de la pluralité d'électrodes et dans lequel le signal de tension alternative de chaque amplificateur à découpage est fourni à l'électrode correspondante de la pluralité d'électrodes ; et
dans lequel le processeur est configuré pour contrôler la puissance produite par chaque électrode.

3. Système selon la revendication 2, dans lequel l'outil médical est un cathéter.

4. Générateur de puissance selon la revendication 1 ou le système selon la revendication 2, dans lequel chaque signal de tension alternative est fourni à la même fréquence.

5. Système selon la revendication 2, dans lequel l'amplitude de chaque signal de tension alternative correspond à la puissance produite par chaque électrode.
